# EUROPÄISCHE PATENTSCHRIFT

(11) **EP 1 422 223 B1**
(45) Veröffentlichungstag und Bekanntmachung des Hinweises auf die Patenterteilung: **14.10.2009**
(21) Anmeldenummer: 03026029.3
(22) Anmeldetag: 12.11.2003
(51) Int. Cl.: C07D 229/00, C08G 18/79

(54) **Herstellung uretdiongruppenhaltiger Polyisocyanate mit Cycloalkylphosphinen als Katalysatoren**
Preparation of polyisocyanates containing uretdione groups with cycloalkylphosphines as catalysts
Préparation de polyisocyanates contenant des groupes uretdione en présence d'un catalyseur cycloalkylphosphine

(30) Priorität: 25.11.2002 DE 10254878
(43) Veröffentlichungstag der Anmeldung: 26.05.2004
(73) Patentinhaber: Bayer MaterialScience AG, 51368 Leverkusen (DE)
(72) Erfinder: Richter, Frank Dr., 51373 Leverkusen (DE); Halpaap, Reinhard Dr., 51519 Odenthal (DE); Laas, Hans-Josef Dr., 51467 Bergisch Gladbach (DE); Hecking, Andreas, 40764 Langenfeld (DE)

(56) Entgegenhaltungen:
- EP-A- 0 495 307
- EP-A- 0 780 377
- EP-A- 1 174 428
- US-A- 3 645 979
- LAAS H J ET AL: "Zur Synthese aliphatischer Polyisocyanate-Lackpolyisocyanate mit Biuret-, Isocyanurat- oder Uretdionstruktur" JOURNAL FUR PRAKTISCHE CHEMIE, CHEMIKER ZEITUNG, WILEY VCH, WEINHEIM, DE, Bd. 336, Nr. 3, 1994, Seiten 185-200, XP000441642 ISSN: 1436-9966

## Beschreibung

Die Erfindung betrifft die Verwendung von Cycloalkylphosphinen als Katalysatoren für die Isocyanatdimerisierung und ein Verfahren zur Herstellung uretdiongruppenhaltiger Polyisocyanate.

Es hat nicht an Versuchen gefehlt, Uretdiongruppen aufweisende aliphatische Polyisocyanate möglichst nebenproduktfrei herzustellen, wobei Katalysatoren eingesetzt werden sollten, deren Selektivität nicht oder nur wenig temperatur- und umsatzabhängig ist.

Uretdiongruppen aufweisende, nebenproduktarme aliphatische Isocyanate auf Basis ggf. verzweigter, linear-aliphatischer Diisocyanate zeichnen sich durch eine besonders niedrige Viskosität aus, Produkte auf Basis cycloaliphatischer Diisocyanate können als abspalterfreie, intern blockierte Vernetzer in Beschichtungssystemen eingesetzt werden.

Tris(dialkylamino)phosphine (DE-A 3 030 513) ggf. in Verbindung mit Cokatalysatoren (DE-A 3 437 635) weisen eine gute Selektivität für die Bildung von Uretdiongruppen (Uretdionselektivität) auf. Ihrer technischen Einsetzbarkeit steht allerdings der schwerwiegende Makel des hohen krebserzeugenden Potenzials ihrer Phosphor(V)-oxide, z.B. Hexamethylphosphorsäuretriamid, entgegen.

DE-A 3 739 549 offenbart die katalytische NCO-Dimerisierung mit 4-Dialkylaminopyridinen, wie z.B. 4-Dimethylaminopyridin (DMAP), wobei allerdings nur im Fall spezieller cycloaliphatischer Isocyanate wie Isophorondiisocyanat (IPDI) die Uretdionbildung selektiv verläuft. Linearaliphatische Isocyanate wie Hexamethylendiisocyanat (HDI) sowie verzweigte, linearaliphatische Isocyanate wie Trimethylhexandiisocyanat (TMDI) und Methylpentandiisocyanat (MPDI) liefern mit DMAP und verwandten Verbindungen hauptsächlich stark gefärbte, heterogene Reaktionsprodukte.

DE-A 1 670 720 offenbart die Herstellung von Uretdiongruppen aufweisenden aliphatischen Polyisocyanaten, wobei als Katalysatoren tertiäre Phosphine mit mindestens einem aliphatischen Substituenten sowie Bortrifluorid und seine Addukte eingesetzt werden. Es wird darauf hingewiesen, dass nur bei niedrigen Umsätzen und Reaktionstemperaturen zwischen 50 und 80°C hohe Anteile an Uretdiongruppen im Produkt erhalten werden können, wobei gleichzeitig Isocyanat-Trimere (Isocyanurate und Iminooxadiazindione) und, insbesondere bei höherer Temperatur, auch andere Nebenprodukte wie Carbodiimide oder Uretonimine gebildet werden. Uretonimine sind ganz besonders störend, da sie bei Lagerung zur Freisetzung von monomerem Isocyanat neigen.

Um die Reaktion bei niedrigen Umsätzen abzubrechen, werden die Phosphinkatalysatoren durch Alkylierung mit Dimethylsulfat (DE-A 1 670 720) oder Toluolsulfonsäuremethylester (EP-A 377 177) desaktiviert und anschließend wird nicht umgesetztes Monomer aus dem Produkt entfernt. Diese Desaktivierungsreaktion erfordert Temperaturen oberhalb 60°C und führt aufgrund ihrer Dauer zu einer Verzögerung des eigentlichen Reaktionsabbruchs der Uretdionbildung und damit insgesamt zu vermehrter Nebenproduktbildung.

Nach der Lehre der DE-A 19 54 093 umgeht man dieses Problem, indem man elementaren Schwefel als Abstopper verwendet. Die Reaktion wird, unabhängig von der Reaktionstemperatur, schlagartig unterbunden. Allerdings ist die benötigte Menge Schwefel schwer zu bestimmen, da bereits während der katalysierten Reaktion partielle Katalysatordeaktivierung eintritt. Überschüssig eingesetzte Mengen des Katalysatorgiftes führen dann zu unvorteilhaften Produkteigenschaften des Polyisocyanats, wie z.B. Trübung, und zu Problemen bei der Wiederverwendung nicht umgesetzten Monomers durch Kontamination mit Schwefel.

Aufgabe der Erfindung war es daher, ein Verfahren zur Herstellung Uretdiongruppen aufweisender Isocyanate bereitzustellen, das gegenüber dem Stand der Technik bei gleichen oder höheren Monomerumsätzen eine höhere Selektivität zur Uretdionbildung (Uretdionselektivität) aufweist, wobei gleichzeitig die Tendenz zur Bildung von Uretoniminen deutlich verringert sein sollte.

Es wurde nun gefunden, dass die erfindungsgemäßen Cycloalkylphosphine mit mindestens einem, direkt an den Phosphor gebundenen, Cycloalkylrest über einen breiteren Temperaturbereich selektiver bezüglich der Uretdionbildung ("Uretdionisierung") ausgehend von organischen Isocyanaten reagieren als bislang hierfür verwendete linearaliphatisch substituierte Phosphine. Darüber hinaus wurde bei Verwendung der erfindungsgemäß zu verwendenden Katalysatoren eine besonders niedrige Uretonimin-Bildungstendenz festgestellt, die sich besonders positiv auf die Lagerungseigenschaften der hergestellten Polyisocyanate auswirkt.

Gegenstand der Erfindung ist die Verwendung von Phosphinen, die mindestens einen, direkt an Phosphor gebundenen Cycloalkylrest aufweisen, als Katalysatoren für die Uretdion-Bildung (Isocyanat-Dimerisierung, "Uretdionisierung").

Erfindüngsgemäß zu verwendende Phosphine sind Phosphine der Formel I : 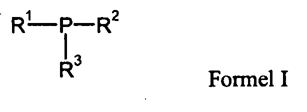 wobei
- R¹: ein ggf. ein- oder mehrfach C₁-C₁₂ alkylsubstituierter Cyclopropyl-, Cyclo- butyl-, Cyclopentyl- oder Cyclohexyl-Rest,
- R², R³: unabhängig voneinander ein ggf. ein- oder mehrfach C₁-C₁₂ alkylsubstituierter Cyclopropyl-, Cyclobutyl-, Cyclopentyl- oder Cyclohexyl-Rest oder ein aliphatischer C₂-C₈-Alkylrest ist.

Beispiele erfindungsgemäß einzusetzender Cycloalkylphosphine sind: Cyclopentyldimethylphosphin, Cyclopentyl-diethylphosphin, Cyclopentyl-di-n-propylphosphin, Cyclopentyl-di-isopropylphosphin, Cyclopentyl-dibutylphosphin, wobei 'Butyl' für alle Isomeren, d.h. n-Butyl, iso-Butyl, 2-Butyl, tert. -Butyl sowie cyclo-Butyl stehen kann, Cyclopentyl-dihexylphosphin (alle isomeren Hexylreste), Cyclopentyl-dioctylphosphin (alle isomeren Octylreste), Dicyclopentyl-methylphosphin, Dicyclopentylethylphosphin, Dicyclopentyl-n-propylphosphin, Dicyclopentyl-isopropylphosphin, Dicyclopentyl-butylphosphin (alle isomeren Butylreste), Dicyclopentyl-hexylphosphin (alle isomeren Hexylreste), Dicyclopentyl-octylphosphin (alle isomeren Octylreste), Tricyclopentylphosphin, Cyclohexyl-dimethylphosphin, Cyclohexyl-diethylphosphin, Cyclohexyl-di-n-propylphosphin, Cyclohexyl-di-isopropylphosphin, Cyclohexyl-dibutylphosphin (alle isomeren Butylreste), Cyclohexyl-dihexylphosphin (alle isomeren Hexylreste), Cyclohexyl-dioctylphosphin (alle isomeren Octylreste), Dicyclohexyl-methylphosphin, Dicyclohexyl-ethylphosphin, Dicyclohexyl-n-propylphosphin, Dicyclohexyl-isopropylphosphin, Dicyclohexyl-butylphosphin (alle isomeren Butylreste), Dicyclohexyl-hexylphosphin (alle isomeren Hexylreste), Dicyclohexyl-octylphosphin (alle isomeren Octylreste), und Tricyclohexylphosphin.

Diese können als Katalysator für die Uretdionbildung einzeln, in beliebigen Mischungen untereinander oder in Mischungen mit anderen primären, sekundären und/oder tertiären Alkyl-, Aralkyl- und/oder Arylphosphinen verwendet werden.

Ein weiterer Gegenstand der Erfindung ist ein Verfahren zur Herstellung uretdiongruppenhaltiger Polyisocyanate, bei dem
a) mindestens ein organisches Isocyanat,
b) ein Katalysator enthaltend mindestens ein Phosphin, das der Formel I entspricht,
c) optional Lösemittel und
d) optional Additive
zur Reaktion gebracht werden und der Katalysator aus b) unmittelbar nach Erreichen eines Umsatzes von 5-90 Mol-% der NCO-Gruppen ohne chemische Deaktivierung vom Reaktionsgemisch abgetrennt wird.

Die Menge des im erfindungsgemäßen Verfahren einzusetzenden Katalysators richtet sich in erster Linie nach der angestrebten Reaktionsgeschwindigkeit und liegt im Bereich 0,01 bis 3 Mol.-%, bezogen auf die Summe der Stoffmengen in Mol des eingesetzten Isocyanates und des Katalysators. Bevorzugt werden 0,05 bis 2 Mol.-% Katalysator eingesetzt.

Der Katalysator b) kann im erfindungsgemäßen Verfahren unverdünnt oder in Lösungsmitteln gelöst eingesetzt werden. Als Lösungsmittel kommen dabei alle Verbindungen in Frage, die nicht mit Phosphinen reagieren wie z.B. aliphatische oder aromatische Kohlenwasserstoffe, Alkohole, Ketone, Ester sowie Ether. Bevorzugt werden die Phosphine im erfindungsgemäßen Verfahren unverdünnt eingesetzt.

Komponente a) sind aliphatische, cycloaliphatische oder araliphatische Di- oder Polyisocyanate einer NCO-Funktionalität ≥ 2.

Besonders bevorzugt ist die Verwendung ggf. verzweigter, ggf. cyclische Reste enthaltender aliphatischer Diisocyanate mit an ein primäres Kohlenstoffatom gebundenen Isocyanatgruppen. Beispiele hierfür sind Butandiisocyanat, Pentandiisocyanat, Hexandiisocyanat, Heptandiisocyanat, Octandiisocyanat, Nonandiisocyanat, Dekandiisocyanat, Undekandiisocyanat und Dodecandiisocyanat, wobei beliebige Isomere der vorstehend genannten Verbindungen zum Einsatz kommen können.

Insbesondere werden Hexamethylendiisocyanat (HDI), Methylpentandiisocyanat (HDI), Trimethylhexandiisocyanat (TMDI), Bis(isocyanatomethyl)cyclohexan (H₆XDI) sowie Norbornandiisocyanat (NBDI) einzeln oder in beliebigen Mischungen untereinander eingesetzt.

Darüber hinaus können Isophorondiisocyanat (IPDI), Bis(isocyanatocyclohexyl)-methan (H₁₂MDI), Bis(isocyanatomethyl)benzol (Xylylendiisocyanat, XDI) und Bis(2-isocyanatoprop-2-yl)benzol (Tetramethylxylylendiisocyanat, TMXDI) im erfindungsgemäßen Verfahren eingesetzt werden.

Das erfindungsgemäße Verfahren wird im Temperaturbereich 0°C bis 120°C, bevorzugt 0°C bis 100°C, besonders bevorzugt 0°C bis 80°C, insbesondere 0°C bis 60°C durchgeführt.

Das erfindungsgemäße Verfahren wird so geführt, dass der Umsatz der NCO-Gruppen von 5 bis 90 Mol.-%, bevorzugt 10 bis 60 Mol.-%, besonders bevorzugt von 10 bis 50 Mol.-% beträgt.

Das Verfahren wird ohne chemische Deaktivierung des Katalysators abgebrochen. Dazu wird unmittelbar nach Erreichen des gewünschten Umsatzes der aktive Katalysator aus der Reaktionsmischung abgetrennt, um eine Weiterreaktion ggf. unter Nebenproduktbildung zu unterbinden.

Gleichzeitig mit oder auch nach der Katalysatorabtrennung kann nicht umgesetztes Restmonomer aus der nach Variante B behandelten Reaktionsmischung abgetrennt werden.

Im erfindungsgemäßen Verfahren können zur Abtrennung nicht umgesetzter Monomere, des Katalysators und/oder anderer unerwünschter Bestandteile aus der Reaktionsmischung alle bekannten Separationstechniken wie z.B. Destillation, Extraktion oder Kristallisation/Filtration verwendet werden. Bevorzugt ist die Destillation, ggf. in der speziellen Ausführungsform der Dünnschichtdestillation. Selbstverständlich können auch Kombinationen zweier oder mehrerer dieser Techniken angewendet werden.

Bevorzugt wird zum Reaktionsabbruch der Katalysator destillativ entfernt, wobei gleichzeitig ggf. nicht umgesetztes Monomer mit entfernt werden kann.

Bevorzugt wird bei der Aufarbeitung das enthaltene Restmonomer destillativ entfernt.

Soll das erfindungsgemäß hergestellte Polyisocyanat noch freies, nicht umgesetztes Monomer enthalten, wie es z.B. für die Weiterverarbeitung zu NCO-blockierten Produkten oder NCO-armen bzw. -freien Polyuretdionhärtern z.B. für den Pulverlackbereich von Interesse ist, so kann nach Reaktionsabbruch (Varianten A und B) auf die Monomerenabtrennung verzichtet werden.

Es ist für die Durchführung des erfindungsgemäßen Verfahrens unerheblich, ob das Verfahren ganz oder teilweise diskontinuierlich oder kontinuierlich durchgeführt wird.

Weiterhin können im erfindungsgemäßen Verfahren zu einem beliebigen Zeitpunkt, in der Polyisocyanatchemie übliche Additive und Stabilisatoren zugesetzt werden. Beispiele sind Antioxidanzien, wie z.B. sterisch gehinderte Phenole (2,6-Di-tert.butylphenol, 4-Methyl-2,6-di-tert.butylphenol), Lichtschutzmittel, wie z.B. HALS-Amine, Triazole etc., schwache Säuren oder Katalysatoren für die NCO-OH-Reaktion wie z.B. Dibutylzinndilaurat (DBTL).

Des weiteren kann es sinnvoll sein, einem aufgearbeiteten Produkt, geringe Mengen eines Katalysatorgiftes zuzusetzen, um die Rückspaltstabilität zu erhöhen und die Neigung zu Nebenproduktbildung bzw. Weiterreaktion der freien NCO-Gruppen, z.B. bei Produktlagerung, zu verringern. Als Katalysatorgifte eignen sich prinzipiell alle vorbeschriebenen Katalysatorgift (DE-A 1670667, 1670720, 1934763, 1954093, 3437635, US4614785) wie Alkylierungsmittel (z.B. Dimethylsulfat, Toluolsufonsäuremethylester), organische oder anorganische Peroxide, Säurechloride sowie Schwefel.

Nach dem erfindungsgemäßen Verfahren hergestellte Produkte auf Basis ggf. verzweigter, linearaliphatischer Di- oder Polyisocyanate, die keine Cycloalkylsubstituenten aufweisen, sind farbhell und haben eine Viskosität < 1000 mPas/23°C. Werden cycloaliphatische und/oder araliphatische Di- oder Polyisocyanate eingesetzt, werden hochviskose bis feste Harze erhalten (Viskosität > 10000 mPas/23°C).

In monomerenarmer Form, d.h. nach Abtrennung von nicht umgesetztem Monomer, weisen die erfindungsgemäßen Produkte einen NCO-Gehalt < 30 Gew.-%, bevorzugt < 25 Gew.-%, auf.

Die nach dem erfindungsgemäßen Verfahren hergestellten Polyisocyanate dienen als Ausgangsmaterialien zur Herstellung von z.B. Formkörpem (ggf. geschäumt), Lacken, Beschichtungsmitteln, Klebstoffen oder Zuschlagstoffen, wobei die enthaltenen freien, nicht uretdionisierten NCO-Gruppen auch ggf. blockiert sein können.

Zur Blockierung der freien, nicht uretdionisierten NCO-Gruppen eignen sich alle dem Fachmann bekannten Methoden. Als Blockierungsmittel können insbesondere Phenole (z.B. Phenol, Nonylphenol, Kresol), Oxime (z.B. Butanonoxim, Cyclohexanonoxim), Lactame (z.B. ε-Caprolactam), sekundäre Amine (z.B. Diisopropylamin), Pyrazole (z.B. Dimethylpyrazol), Imidazole, Triazole oder Malon- und Essigsäureester verwendet werden.

Die nach dem erfindungsgemäßen Verfahren hergestellten, weitgehend nebenproduktfreien, Uretdiongruppen aufweisenden Polyisocyanate können insbesondere zur Herstellung von Ein- und Zweikomponenten-Polyurethanlacken ggf. in Mischungen mit anderen Di- oder Polyisocyanaten des Standes der Technik, wie Biuret-, Urethan-, Allophanat-, Isocyanurat-, sowie Iminooxadiazindiongruppen enthaltenden Di- oder Polyisocyanaten eingesetzt werden.

Ebenfalls besonders bevorzugt ist die Verwendung der erfmdungsgemäß hergestellten Polyisocyanate auf Basis ggf. verzweigter, linearaliphatischer Isocyanate als Reaktivverdünner zur Viskositätserniedrigung höherviskoser Polyisocyanat-Harze.

Zur Umsetzung der erfindungsgemäß hergestellten Polyisocyanate zum Polyurethan können alle Verbindungen mit mindestens zwei isocyanatreaktiven Funktionalitäten einzeln oder in beliebigen Mischungen untereinander (isocyanatreaktives Bindemittel) eingesetzt werden.

Bevorzugt ist die Verwendung eines oder mehrerer, in der Polyurethanchemie an sich bekannter, isocyanatreaktiver Bindemittel wie Polyhydroxyverbindungen oder Polyamine. Als Polyhydroxyverbindungen werden besonders bevorzugt Polyester-, Polyether-, Polyacrylat- und/oder Polycarbonsäure-Polyole, ggf. auch unter Zusatz niedermolekularer, mehrwertiger Alkohole eingesetzt.

Das Äquivalentverhältnis zwischen nicht uretdionisierter Isocyanatgruppe, die ggf. auch blockiert sein kann, und isocyanatreaktiver Funktionalität des isocyanatreaktiven Bindemittels, wie z.B. OH-, NH- oder COOH, liegt von 0,8 bis 3, vorzugsweise 0,8 bis 2.

Möglich ist der Einsatz eines Überschusses an isocyanatreaktivem Bindemittel, da die Spaltung des Uretdionringes ggf. bei erhöhter Temperatur und/oder Katalysatorzusatz zur Freisetzung weiterer NCO-Gruppen führt, die mit dem Überschuss an isocyanatreaktiven Funktionalitäten reagieren können. Dadurch erhöht sich die Netzwerkdichte des gebildeten Polymers und dessen Eigenschaften werden vorteilhaft beeinflusst.

Für die Beschleunigung der Vernetzungsreaktion der erfindungsgemäß hergestellten Polyisocyanate mit dem isocyanatreaktiven Bindemittel können alle aus der Polyurethanchemie bekannten Katalysatoren verwendet werden. Beispielweise können Metallsalze wie Dibutylzinn-IV-dilaurat, Zinn-II-bis(2-ethylhexanoat), Wismut-III-tris(2-ethylhexanoat), Zink-II-bis(2-ethylhexanoat) oder Zinkchlorid sowie tertiäre Amine wie 1,4-Diazabicyclo(2,2,2)oktan, Triethylamin oder Benzyldimethylamin verwendet werden.

Bei der Formulierung werden das erfindungsgemäß hergestellte, ggf. blockierte Polyisocyanat, das isocyanatreaktive Bindemittel, Katalysator(en) und ggf. die üblichen Zusätze wie Pigmente, Füllstoffe, Additive, Verlaufshilfsmittel, Entschäumer und/oder Mattierungsmittel miteinander auf einem üblichen Mischaggregat wie z.B. einer Sandmühle, ggf. unter Verwendung von Lösungsmitteln, vermischt und homogenisiert.

Als Lösungsmittel geeignet sind alle an sich bekannten üblichen Lacklösemittel wie z.B. Ethyl- und Butylacetat, Ethylen- oder Propylenglykolmono-methyl-, -ethyl- oder - propyletheracetat, 2-Butanon, 4-Methyl-2-pentanon, Cyclohexanon, Toluol, Xylol, Solventnaphtha, N-Methylpyrrolidon etc.

Die Beschichtungsmittel können in Lösung oder aus der Schmelze sowie ggf. in fester Form (Pulverlacke) nach den üblichen Methoden wie z.B. Streichen, Rollen, Gießen, Spritzen, Tauchen, dem Wirbelsinterverfahren oder durch elektrostatische Sprühverfahren auf dem zu beschichtenden Gegenstand appliziert werden.

Als Substrate eignen sich sämtliche bekannten Werkstoffe, insbesondere Metalle, Holz, Kunststoffe und Keramik.

### Beispiele:

Alle Prozentangaben sind, soweit nicht anders vermerkt, als Gewichtsprozent (Gew.-%) zu verstehen.

Die Ermittlung des NCO-Gehaltes der in den Beispielen und Vergleichsbeispielen beschriebenen Harze erfolgte durch Titration gemäß DIN 53 185.

Die dynamischen Viskositäten wurden bei 23°C mit einem Rotationsviskosimeter (ViscoTester® 550, Thermo Haake GmbH, D-76227 Karlsruhe) bestimmt. Durch Messungen bei unterschiedlichen Schergeschwindigkeiten wurde sichergestellt, dass das Fließverhalten der beschriebenen erfindungsgemäß hergestellten Polyisocyanate wie auch das der Vergleichsprodukte dem idealer Newtonscher Flüssigkeiten entspricht. Die Angabe der Schergeschwindigkeit kann deshalb entfallen.

Die Angabe 'Mol.-%' bzw. des molaren Verhältnisses unterschiedlicher Strukturtypen zueinander basiert auf NMR-spektroskopischen Messungen. Sie bezieht sich immer, wenn nicht anders angegeben, auf die Summe der durch die Modifizierungsreaktion (Oligomerisierung) aus den vorher freien NCO-Gruppen des zu modifizierenden Isocyanates gebildeten Strukturtypen. Die ¹³C-NMR-Messungen erfolgten an ca. 50 Gew.-%-igen Proben in trockenem CDCl₃ bzw. ca. 80 Gew.-%-igen Proben in D₆-DMSO bei einer Protonenfrequenz von 400 bzw. 700 MHz (¹³C-NMR: 100 bzw. 176 MHz, relaxation delay: 4 sec, 2000 scans; Spektrometer: DPX 400, AVC 400 bzw. DRX 700, Bruker GmbH, D-76287 Rheinstetten). Als Referenz für die ppm-Skala wurden geringe Mengen von Tetramethylsilan im Lösungsmittel mit einer ¹³C-chem. Verschiebung von 0 ppm bzw. das Lösungsmittel selbst mit einer Verschiebung von 77,0 ppm (CDCl₃) bzw. 43,5 ppm (D₆-DMSO) gewählt.

Soweit nicht anders angegeben, wurden die Reaktionen mit HDI als Edukt durchgeführt.

### Beispiel 1:

Jeweils 10 g frisch destilliertes, entgastes HDI wurden in mit Septen verschlossenen Glasgefäßen unter Stickstoff in Gegenwart der in Tabelle 1 angegebenen Mengen des Katalysators bei den angegebenen Temperaturen gerührt (Magnetrührer), wobei in regelmäßigen Abständen der Fortgang der Reaktion durch Messung des Brechungsindex' (bei 20°C und der Frequenz des Lichtes der D-Linie des Natriumspektrums, n_{D}²⁰) der Reaktionsmischung (Rohware) überprüft wurde (Start = kein Umsatz = n_{D}²⁰ des reinen HDI = 1,4523).

**Tabelle 1: Reaktionsparameter**

| Temperatur | TBP | CHDHP | DCPBP | TCPP |
|---|---|---|---|---|
| [°C] | [Mol.-%]* | [Mol.-%]* | [Mol.-%]* | [Mol.-%]* |
| 40 | 0,18 | 0,60 | 0,70 | 1,14 |
| 60 | 0,18 | 0,80 | 0,73 | 1,13 |
| 80 | 0,25 | 0,50 | 0,46 | 1,06 |
| 100 | 0,30 | 0,48 | 0,47 | 1,06 |
| 120 | 0,31 | 0,56 | 0,55 | 1,04 |

| | | | | |
|---|---|---|---|---|
| *: bezogen auf eingesetzte Menge HDI | | | | |

### Abkürzungen:

- TBP:: Tri-n-butylphosphin (=> Vergleichsversuche)
- CHDHP:: Cyclohexyl-di-n-hexylphosphin (=> erfindungsgemäßer Versuch)
- DCPBP:: Dicyclopentyl-butylphosphin (=> erfindungsgemäßer Versuch)
- TCPP:: Tricyclopentylphosphin (=> erfindungsgemäßer Versuch)

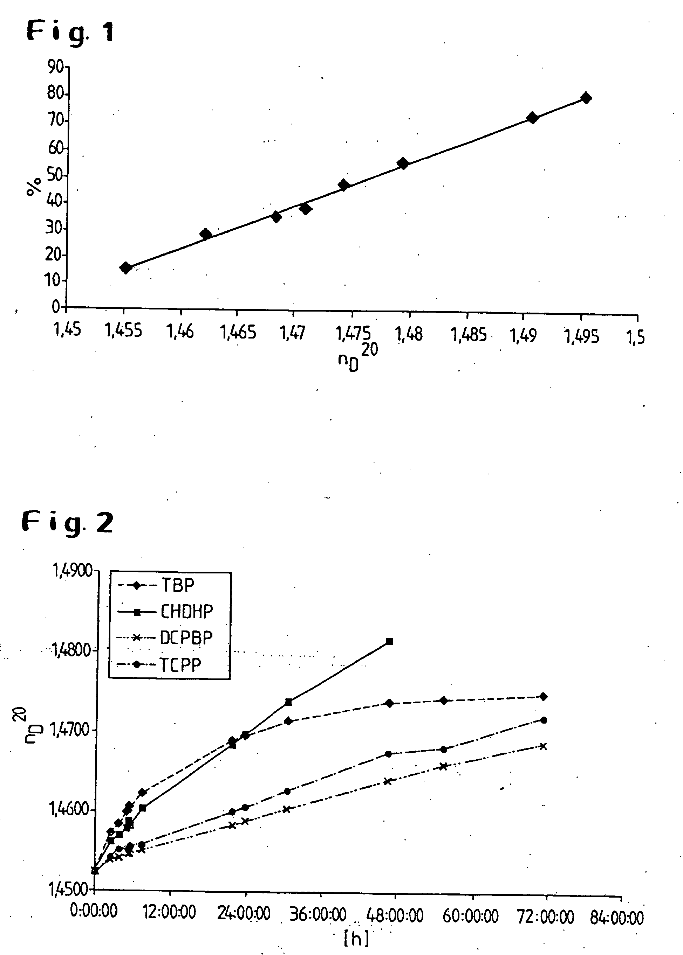

Durch Aufnahme von Kalibrierkuven anhand größerer Ansätze, die bei unterschiedlichen Umsetzungsgraden destillativ aufgearbeitet wurden, ist der n_{D}²⁰-Wert der Rohware zur Harzausbeute [%], im weiteren kurz Ausbeute, für verschiedene Katalysatoren in Beziehung gesetzt worden. Im Bereich bis etwa 80 % Ausbeute wurde unabhängig von Katalysator und Reaktionstemperatur eine annähernd lineare Beziehung beider Größen zueinander erhalten (Figur 1), so dass sich durch Brechungsindex-Messung stets die Harzausbeute in-situ bestimmen lässt.

Tri-n-butylphosphin (TBP) bewirkt bei gleicher (molarer) Konzentration eine höhere Reaktionsgeschwindigkeit im Vergleich zu den erfindungsgemäß zu verwendenden Katalysatoren. Letztere sind mit zunehmender Anzahl an P-gebundenen Cycloalkylgruppen weniger aktiv, aber dafür deutlich selektiver bezüglich der Uretdionbildung. Daher sind die verwendeten TBP-Mengen stets niedriger als die der Cycloalkylphosphine um die Reaktionsgeschwindigkeiten vergleichbar zu halten. Hinzu kommt, dass bei Verwendung von TBP nach einer anfangs relativ zügigen Reaktion schnell eine Desaktivierung des Katalysators einsetzt, erkennbar am immer geringer werdenden Anstieg der Zeit-Ausbeute-Kurve mit Voranschreiten der Reaktion. Mit den Cycloalkyl-substituierten Phosphinen hingegen wird auch noch bei hohen Ausbeuten eine wesentlich gleichmäßigere Reaktionsführung erzielt (Figur 2). Die Menge des jeweils verwendeten Katalysators richtete sich im Beispiel 1 lediglich nach der angestrebten Reaktionsgeschwindigkeit. Die Katalysatorkonzentration hat - in den o.g. Grenzen - keinen detektierbaren Einfluss auf die Selektivität der Reaktion, wie anhand von Vergleichsversuchen mit höherer TBP-Konzentration bei verschiedenen Temperaturen belegt wurde.

Zur Überprüfung der Temperatur- und Katalysatorabhängigkeit der Uretdionselektivität wurden bei Erreichen von n_{D}²⁰-Werten von 1,4550, 1,4670, 1,4740 bzw. 1,4830 entsprechend Harzausbeuten von ca. 15, 35, 45 und 60 % (vgl. Figur 1) jeweils 0,5 ml Reaktionsmischung unter Stickstoff entnommen, in ein NMR-Röhrchen überführt und nach Zugabe von 0,15 ml einer 1 %-igen Lösung von Benzoylchlorid in D₆-DMSO (zur Deaktivierung des Phosphins) ¹³C-NMR-spektroskopisch untersucht.

Zur besseren Übersichtlichkeit der Selektivitäten wurde die Größe U/T als das molare Verhältnis der Uretdionstrukturen zur Summe der beiden Trimerstrukturen (Isocyanurat und Iminooxadiazindion) definiert. Die U/T-Werte bei den o.g. Ausbeuten (ca. 15, 35, 45 bzw. 60 Gew.-%) sind in den Tabellen 2-5 dargestellt.

**Tabelle 2: U/T-Selektivitäten für ca. 15 Gew.-% Ausbeute in Abh. von Katalysator und Reaktionstemperatur**

| Temperatur [°C] | U/T(TBP) | U/T(CHDHP) | U/T(DCPBP) | U/T(TCPP) |
|---|---|---|---|---|
| 40 | 4,0 | 4,2 | 7,4 | 10,2 |
| 60 | 4,9 | 5,3 | 7,5 | 32,9 |
| 80 | 6,8 | 7,2 | 13,4 | 37,3 |
| 100 | 7,2 | 12,3 | 11,4 | 41,7 |
| 120 | Versuchsprodukte unbrauchbar aufgrund zu hoher Uretoniminanteile, vgl. Tabelle 6 | | | |

**Tabelle 3: U/T-Selektivitäten für ca. 35 Gew.-% Ausbeute in Abh. von Katalysator und Reaktionstemperatur**

| Temperatur [°C] | U/T(TBP) | U/T(CHDHP) | U/T(DCPBP) | U/T(TCPP) |
|---|---|---|---|---|
| 40 | 3,2 | 3,6 | 5,7 | 8,0 |
| 60 | 3,4 | 4,3 | 5,8 | 11,3 |
| 80 | 3,4 | 4,1 | 4,8 | 8,1 |
| 100 | 3,2 | 2,7 | 2,8 | 4,1 |
| 120 | Versuchsprodukte unbrauchbar aufgrund zu hoher Uretoniminanteile, vgl. Tabelle 7 | | | |

**Tabelle 4: U/T-Selektivitäten für ca. 45 Gew.-% Ausbeute in Abh. von Katalysator und Reaktionstemperatur**

| Temperatur [°C] | U/T(TBP) | U/T(CHDHP) | U/T(DCPBP) | U/T(TCPP) |
|---|---|---|---|---|
| 40 | 2,8 | 3,3 | 4,8 | 6,9 |
| 60 | 2,7 | 3,8 | 4,9 | 8,2 |
| 80 | 2,4 | 3,1 | 3,5 | 5,1 |
| 100 | 2,5 | 1,7 | 1,9 | 2,1 |
| 120 | Versuchsprodukte unbrauchbar aufgrund zu hoher Uretoniminanteile, vgl. Tabelle 8 | | | |

**Tabelle 5: U/T-Selektivitäten für ca. 60 Gew.-% Ausbeute in Abh. von Katalysator und Reaktionstemperatur**

| Temperatur | U/T(TBP) | U/T(CHDHP) | U/T(DCPBP) | U/T(TCPP) |
|---|---|---|---|---|
| 40 | 2,2 | 2,8 | 3,5 | 5,3 |
| 60 | 1,6 | 3,0 | 3,6 | 5,7 |
| 80 | 1,3 | 2,1 | 2,5 | 3,1 |
| 100 | 1,9 | 1,0 | 1,2 | 1,0 |
| 120 | Versuchsprodukte unbrauchbar aufgrund zu hoher Uretoniminanteile, vgl. Tabelle 9 | | | |

**Tabelle 6: Mol.-% gebildetes Uretonimin im Reaktionsprodukt für ca. 15 Gew.-% Ausbeute in Abh. von Katalysator und Reaktionstemperatur**

| Temperatur [°C] | TBP | CHDHP | DCPBP | TCPP |
|---|---|---|---|---|
| 40 | n.n. | n.n. | n.n. | n.n. |
| 60 | n.n. | n.n. | n.n. | n.n. |
| 80 | n.n. | n.n. | n.n. | n.n. |
| 100 | n.n. | n.n. | n.n. | n.n. |
| 120 | 14,5 | 5,9 | 7,1 | n.n. |

**Tabelle 7: Mol.-% gebildetes Uretonimin im Reaktionsprodukt für ca. 35 Gew.-% Ausbeute in Abh. von Katalysator und Reaktionstemperatur**

| Temperatur [°C] | TBP | CHDHP | DCPBP | TCPP |
|---|---|---|---|---|
| 40 | n.n. | n.n. | n.n. | n.n. |
| 60 | n.n. | n.n. | n.n. | n.n. |
| 80 | 1,1 | n.n. | n.n. | n.n. |
| 100 | 19,3 | 5,3 | n.n. | n.n. |
| 120 | 36,6 | 14,4 | 12,0 | n.n. |

**Tabelle 8: Mol.-% gebildetes Uretonimin im Reaktionsprodukt für ca. 45 Gew.% Ausbeute in Abh. von Katalysator und Reaktionstemperatur**

| Temperatur [°C] | TBP | CHDHP | DCPBP | TCPP |
|---|---|---|---|---|
| 40 | n.n. | n.n. | n.n. | n.n. |
| 60 | n.n. | n.n. | n.n. | n.n. |
| 80 | 4,2 | 1,6 | n.n. | n.n. |
| 100 | 32,8 | 6,6 | 6,0 | n.n. |
| 120 | 47,7 | 18,7 | 14,5 | 2,5 |

**Tabelle 9: Mol.% gebildetes Uretonimin im Reaktionsprodukt für ca. 60 Gew.-% Ausbeute in Abh. von Katalysator und Reaktionstemperatur**

| Temperatur [°C] | TBP | CHDHP | DCPBP | TCPP |
|---|---|---|---|---|
| 40 | n.n. | n.n. | n.n. | n.n. |
| 60 | n.n. | n.n. | n.n. | n.n. |
| 80 | 8,9 | 2,1 | 1,9 | n.n. |
| 100 | 53,1 | 8,5 | 7,3 | 1,5 |
| 120 | 64,3 | 25,0 | 18,2 | 3,2 |

### Abkürzungen:

- TBP:: Tri-n-butylphosphin (=> Vergleichsversuche)
- CHDHP:: Cyclohexyl-di-n-hexylphosphin (=> erfindungsgemäßer Versuch)
- DCPBP:: Dicyclopentyl-butylphosphin (=> erfindungsgemäßer Versuch)
- TCPP:: Tricyclopentylphosphin (=> erfindungsgemäßer Versuch)
- n.n.:: durch ¹³C-NMR-Spektroskopie nicht detektiert

Wie man aus den vorstehenden Tabellen entnehmen kann, ist die Uretdionselektivität der erfindungsgemäßen Katalysatoren bei gegebener Ausbeute und niedrigem Uretonimingehalt der Produkte in der Regel höher als bei Tri-n-butylphosphin (TBP).

Bemerkenswert ist auch bei höheren Temperaturen die besonders niedrige Tendenz zur Uretonimin-Bildung bei Verwendung der erfindungsgemäßen Katalysatoren, die stets signifikant geringer als bei Verwendung von TBP ist.

### Beispiele 2:

Jeweils 1500 g HDI wurden in einem Rührgefäß bei 60 °C eine Stunde unter Rühren im Vakuum (0,5 mbar) von gelösten Gasen befreit, anschließend mit Stickstoff belüftet und nach Abkühlen auf 40°C 2-A: mit 6,0 g TBP (=> Vergleichsversuch) bzw. 2-B: mit 21,0 g DCPBP (=> erfindungsgemäßer Versuch) versetzt.

Anschließend wurde weiter bei 40°C gerührt, wobei der Anstieg des Umsatzes refraktrometrisch verfolgt wurde. Bei Erreichen eines n_{D}²⁰ von ca. 1,4630 (Sollumsatz) wurde destillativ in einem Kurzwegverdampfer mit vorgeschaltetem Vorverdampfer bei 0,3 mbar und einer Temperatur des Heizmediums von 130°C (Vorverdampfer) bzw. 140°C (Dünnschichtverdampfer) aufgearbeitet. Das Destillat wurde anschließend unter Stickstoff mit frischem, entgastem HDI auf die Ausgangsmenge aufgefüllt, erneut bei 40°C bis zum Erreichen des o.g. Brechungsindex' gerührt und anschließend aufgearbeitet wie zuvor beschrieben. Diese Sequenz wurde insgesamt 7 mal wiederholt. Die jeweils nötige Reaktionszeit ist Tabelle 10, die Daten der isolierten Harze sind Tabelle 11 zu entnehmen.

**Tabelle 10: Reaktionszeiten (hh:mm) der Versuche 2-A und -B bis Sollumsatz**

| Versuch | Katalysator | 1 | 2 | 3 | 4 | 5 | 6 | 7 | 8 |
|---|---|---|---|---|---|---|---|---|---|
| 2A | TBP | 04:01 | 05:08 | 03:10 | 04:25 | 03:38 | 09:40 | 12:27 | 14:24 |
| 2B | DCPBP | 14:09 | 12:48 | 13:13 | 12:31 | 13:03 | 12:00 | 15:18 | 16:37 |

**Tabelle 11: Daten (Durchschnittswerte der 8 Versuche) der Harze aus den Versuchen: 6-A: Vergleichsversuch, 6-B: erfindungsgemäße Umsetzung**

| Versuch | Katalysator | Ausbeute [%] | NCO-Gehalt [%] | Viskosität [mPas] | Farbzahl [APHA] | freies HDI [%] | U/T |
|---|---|---|---|---|---|---|---|
| 2A | TBP | 27,9 | 22,3 | 130 | 53 | 0,12 | 2,8 |
| 2B | DCPBP | 30,0 | 22,2 | 82 | 35 | 0,07 | 4,1 |

Bei Verwendung des erfindungsgemäßen Katalysators DCPBP wurde eine wesentlich gleichmäßigere Reaktionsführung beobachtet, als bei Verwendung von TBP. Dies ist für die praktische Einsetzbarkeit der Phosphine in einem Prozess mit kontinuierlicher Fahrweise von entscheidender Bedeutung. Darüber hinaus wurden im erfindungsgemäßen Verfahren bei höherer Ausbeute Harze mit niedrigerer Viskosität infolge eines höheren Uretdionanteils erhalten. Darüber hinaus zeichnen sich die erfindungsgemäß hergestellten Harze durch einen niedrigeren HDI-Gehalt aus.

## Patentansprüche

1. Verfahren zur Herstellung uretdiongruppenhaltiger Polyisocyanate, bei dem
a) aliphatische, cycloaliphatische oder araliphatische Di- oder Polyisocyanate einer NCO-Funktionalität ≥ 2 in Anwesenheit
b) eines Katalysators enthaltend ein Phosphin, das der Formel (I) entspricht 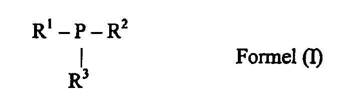 wobei
R¹ ein ggf. ein- oder mehrfach C₁-C₁₂ alkylsubstituierter Cyclopropyl-, Cyclobutyl-, Cyclopentyl- oder Cyclohexyl-Rest ist und
R², R³ unabhängig voneinander ein ggf. ein- oder mehrfach C₁-C₁₂ alkylsubstituierter Cyclopropyl-, Cyclobutyl-, Cyclopentyl- oder Cyclohexyl-Rest oder ein aliphatischer C₂-C₈-Alkylrest sind.
c) optional Lösemittel und
d) optional Additive
zur Reaktion gebracht werden und der Katalyator aus b) unmittelbar nach Erreichen eines Umsatzes von 5-90 Mol.-% der NCO-Gruppen ohne chemische Deaktivierung von Reaktionsgemisch abgetrennt wird.

2. Verfahren gemäß Anspruch 1, **dadurch gekennzeichnet, dass** als organische Isocyanate Hexamethylendiisocyanat (HDI), Methylpentandiisocyanat (MPDI), Trimethylhexandiisocyanat (TMDI), Bis(isocyanatomethyl)cyclohexan (H₆XDI) sowie Norbornandiisocyaat (NBDI) oder deren beliebige Mischungen eingesetzt werden.

3. Verfahren gemäß Anspruch 1 oder 2, **dadurch gekennzeichnet, dass** die Katalysatorkomponente b) ausschließlich aus Phosphinen mit mindestens einem direkt an Phosphor gebundenen cycloaliphatischen Rest besteht.

## Claims

1. Process for the preparation of polyisocyanates containing uretdione groups, in which
a) aliphatic, cycloaliphatic or araliphatic di- or polyisocyanates having an OH functionality of ≥ 2 are reacted in the presence of
b) a catalyst containing a phosphine which corresponds to the formula (I) 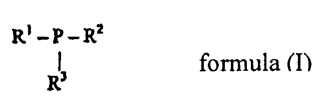 wherein
R¹ is an optionally mono- or poly-C₁-C₁₂-alkyl-substituted cyclopropyl, cyclobutyl, cyclopentyl or cyclohexyl radical and
R², R³ independently of each other are an optionally mono- or poly-C₁-C₁₂-alkyl-substituted cyclopropyl, cyclobutyl, cyclopentyl or cyclohexyl radical or an aliphatic C₂-C₈-alkyl radical,
c) optionally solvents and
d) optionally additives
and the catalyst from b) is separated off from the reaction mixture, without chemical deactivation, immediately after a conversion of 5 - 90 mol% of the NCO groups has been reached.

2. Process according to claim 1, **characterized in that** hexamethylenediisocyanate (HDI), methylpentane-diisocyanate (MPDI), trimethylhexane-diisocyanate (TMDI), bis(isocyanatomethyl)cyclohexane (H₆XDI) and norbomane-diisocyanate (NBDI) or any desired mixtures thereof are employed as organic isocyanates.

3. Process according to claim 1 or 2, **characterized in that** the catalyst component b) comprises exclusively phosphines with at least one cycloaliphatic radical bonded directly to phosphorus.

## Revendications

1. Procédé pour la préparation de polyisocyanates contenant des groupes urètdiones, dans lequel on fait réagir
a) des di- ou polyisocyanates aliphatiques, cycloaliphatiques ou araliphatiques d'une fonctionnalité NCO ≥ 2 en présence
b) d'un catalyseur contenant une phosphine, laquelle correspond à la formule (I) 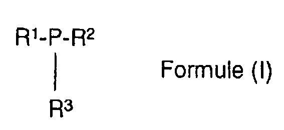 dans laquelle
R¹ représente un reste cyclopropyle, cyclobutyle, cyclopentyle ou cyclohexyle éventuellement une ou plusieurs fois C₁-C₁₂ alkyle-substitué et
R², R³ sont indépendamment l'un de l'autre un reste cyclopropyle, cyclobutyle, cyclopentyle ou cyclohexyle éventuellement une ou plusieurs fois C₁-C₁₂ alkyle-substitué ou un reste C₂-C₈ alkyle aliphatique.
c) d'un solvant facultatif et
d) d'additifs facultatifs
et le catalyseur de b) est, immédiatement après avoir atteint une conversion de 5-90 % en mole des groupes NCO, séparé du mélange réactionnel sans désactivation chimique.

2. Procédé selon la revendication 1, **caractérisé en ce que** l'on utilise comme isocyanates organiques le diisocyanate d'hexaméthylène (HDI), le diisocyanate de méthylpentane (MPDI), le diisocyanate de triméthylhexane (PMDI), le bis(isocyanatométhyl)cyclohexane (H₆XDI) ainsi que le diisocyanate de norbornane (NBDI) ou des mélanges quelconques de ceux-ci.

3. Procédé selon la revendication 1 ou 2, **caractérisé en ce que** le constituant de catalyseur b) est exclusivement constitué de phosphines avec au moins un reste cycloaliphatique directement lié au phosphore.
